Europäisches Patentamt

European Patent Office

Office européen des brevets

(11) Numéro de publication: **0 371 871**
**A1**

## (12) DEMANDE DE BREVET EUROPEEN

(21) Numéro de dépôt: **89403287.9**

(51) Int. Cl.⁵: **A61F 13/15**

(22) Date de dépôt: **28.11.89**

(30) Priorité: **30.11.88 FR 8815715**

(43) Date de publication de la demande:
**06.06.90 Bulletin 90/23**

(84) Etats contractants désignés:
**AT BE CH DE ES FR GB GR IT LI LU NL SE**

(71) Demandeur: **PEAUDOUCE**
**59, Rue de la Vignette**
**F-59126 Linselles(FR)**

(72) Inventeur: **Leroy, André**
**1, Allée des Glycines**
**F-59420 Mouvaux(FR)**
Inventeur: **Deleu, Bernard**
**6, rue Jules Massenet**
**F-59126 Linselles(FR)**
Inventeur: **Naze, Alain**
**44, Chaussée de la Gardie-Dieu**
**B-7791 Bas-Warneton(BE)**

(74) Mandataire: **Casalonga, Axel et al**
**BUREAU D.A. CASALONGA - JOSSE**
**Morassistrasse 8**
**D-8000 München 5(DE)**

(54) **Couche-culotte à élastiques d'entre-jambes.**

(57) Couche-culotte à jeter après usage comprenant une feuille externe (1) imperméable aux liquides, une feuille interne (2) perméable aux liquides, un matelas absorbant (3), des éléments élastiques (18) s'étendant à l'état allongé le long des bords longitudinaux opposés du matelas absorbant, et des moyens (11) de fermeture de la couche-culotte autour de la taille de l'utilisateur.

La couche-culotte comprend, en outre, deux rubans longitudinaux (14) s'étendant à l'état tendu sur toute la longueur de la couche-culotte, le long des bords longitudinaux opposés du matelas (3), entre les deux feuilles (1, 2) chacun des rubans étant fixé au moins le long de son bord longitudinal extérieur (15) et le long de ses deux bords transversaux à la feuille externe (1) et le long de ses deux bords longitudinaux opposés (16) et le long de ses deux bords transversaux à la feuille interne (2), et les éléments élastiques (18) sont fixés à l'état allongé sur les rubans (14) entre les deux bords longitudinaux opposés (15, 16) de ces derniers.

Application : aux articles d'hygiène pour enfants et adultes.

FIG.6

## COUCHE-CULOTTE A ELASTIQUES D'ENTRE-JAMBES.

La présente invention se rapporte à une couche-culotte à jeter après usage, ou un article d'hygiène analogue, utilisable pour des enfants ou des adultes, comprenant un matelas absorbant, une feuille externe imperméable aux liquides, une feuille interne perméable aux liquides, le matelas absorbant étant disposé entre lesdites deux feuilles, des éléments élastiques s'étendant à l'état élastiquement allongé le long des deux bords longitudinaux opposés du matelas absorbant, et des moyens de fermeture de la couche-culotte autour de la taille de l'utilisateur.

Sur les couches-culottes connues de ce type, les éléments élastiques longitudinaux destinés à appliquer la couche-culotte contre les cuisses sont généralement fixés à la face interne de la feuille externe imperméable, à l'extérieur des bords longitudinaux de la couche-culotte. Ces dispositions présentent un certain nombre d'inconvénients.

D'une part, la fonction des éléments élastiques de plaquer la couche-culotte contre les cuisses de l'utilisateur peut être gênée par le matelas absorbant disposé entre eux notamment lorsque le matelas est épais et/ou rigide, ce qui peut conduire à un baillement préjudiciable à l'étanchéité dans cette zone particulièrement exposée aux fuites. De plus, la couche-culotte peut avoir un aspect "gonflé" à l'endroit du matelas absorbant, entre les éléments élastiques (aspect dit en "gant de boxe"). Par ailleurs, lorsque, comme c'est généralement le cas, les couches-culottes sont fabriquées en continu dans le sens longitudinal et les éléments élastiques sont posés en continu après avoir été encollés par intervalles et sont ensuite sectionnés dans les intervalles non encollés de manière que ces sections non encollées se contractent librement, il est nécessaire de prévoir, entre les deux feuilles des couches-culottes, des zones non encollées pour permettre cette libre contraction des éléments élastiques, ces zones non encollées pouvant ensuite donner à des fuites de matière constitutive du matelas et/ou de liquide absorbé par le matelas. Enfin, sur les couches-culottes comportant un matelas absorbant en forme de sablier, avec des échancrures latérales dans la zone dite d'entre-jambes, les éléments élastiques longitudinaux ne sont fixés à la feuille externe imperméable que sur la longueur de ces échancrures, ce qui fait que lorsque la couche-culotte est posée sur l'utilisateur, le plaquage contre les cuisses est limité à une partie seulement de la circonférence des cuisses, ce qui peut donner lieu à des fuites dans la partie restante.

On a certes déjà tenté de remédier à certains des inconvénients précités. Par les demandes de brevets européens n° 219 326, 243 013 et 251 332, il est connu de fixer les éléments élastiques longitudinaux non pas sur la feuille externe imperméable disposée à plat, mais sur des "volets" latéraux faisant saillie vers le haut et ayant une certaine liberté de mouvement par rapport à la feuille externe et, par conséquent, par rapport au matelas absorbant. Ces dispositions entraînent cependant une complication considérable de la fabrication des couches-culottes, ce qui implique un prix de revient élevé incompatible avec la destination de ces produits (produits du type à jeter après usage).

Par ailleurs, il est par exemple connu par la demande de brevet européen n° 0 270 979 d'éliminer les risques de fuites par les zones non collées prévues entre les deux feuilles d'une couche-culotte à éléments élastiques longitudinaux, par la prévision de rubans longitudinaux recouvrant les éléments élastiques et fixés à la feuille externe de part et d'autre des éléments élastiques, sur toute la longueur de la couche-culotte. Cela ne remédie cependant pas aux autres inconvénients précités relatifs aux éléments élastiques fixés à la feuille externe.

Il est également connu, par la demande de brevet européen n° 0 149 999, pour fabriquer une couche-culotte, d'utiliser une feuille externe d'une largeur supérieure à la largeur de la couche-culotte, de replier cette feuille sous la forme de deux rabats latéraux sur le dessus du matelas absorbant, et de fixer des éléments élastiques longitudinaux sur lesdits rabats. Les rabats qui procurent aux éléments élastiques une certaine liberté par rapport au matelas absorbant et également par rapport à la partie restante (pli inférieur) de la feuille externe sont fixés, à leurs extrémités longitudinales, au pli inférieur de la feuille externe, mais sont libres sur la partie restante de leur longueur, ce qui compromet leur position correcte sous la traction des éléments élastiques qui déforment les rabats.

Il apparaît donc que les tentatives faites jusqu'à présente pour remédier aux inconvénients des couches-culottes usuelles à éléments élastiques longitudinaux fixés à l'état allongé sur la feuille externe imperméable ne donnent pas entière satisfaction.

La présente invention a pour objet une couche-culotte répondant mieux aux impératifs imposés à de tels articles. L'invention a pour objet une couche-culotte qui, tout en étant d'une structure et d'une fabrication simples, permet aux éléments élastiques d'assurer un plaquage parfait contre les cuisses de l'utilisateur sans que ce plaquage ne soit gêné ou contrarié par la présence du matelas absorbant entre les éléments élastiques. L'invention a également pour objet une couche-culotte de fabrication simple éliminant ou pour le moins réduisant les risques de fuites dus à la présence, entre les deux feuilles, de zones non encollées pour permettre la contraction des parties non encollées des éléments élastiques longitudinaux. Par ailleurs, l'invention a pour objet une couche-culotte du type à matelas absorbant en forme de sablier sur laquelle les éléments élastiques peuvent assurer un plaquage contre les cuisses de l'utilisateur sur tout le pourtour des cuisses et notamment sur une longueur supérieure à la longueur des échancrures latérales du matelas absorbant.

La couche-culotte conforme à l'invention du type à jeter après usage comprend une feuille externe imperméable aux liquides et une feuille interne perméable aux liquides, les deux feuilles de forme allongée ayant pratiquement les mêmes dimensions et étant superposées. La couche-culotte comprend en outre un matelas absorbant de forme allongée ayant une longueur et une largeur plus faibles que celles desdites feuilles et disposé entre lesdites feuilles de manière que ses bords soient en retrait par rapport aux bords desdites feuilles, les deux feuilles étant reliées entre elles autour du matelas. La couche-culotte comprend, par ailleurs, des éléments élastiques s'étendant à l'état allongé le long des bords longitudinaux opposés du matelas absorbant. Enfin, la couche-culotte comprend des moyens de fermeture de la couche-culotte autour de la taille de l'utilisateur. Selon l'invention, la couche-culotte comprend, en outre, deux rubans longitudinaux s'étendant à l'état tendu sur toute la longueur de la couche-culotte, le long des bords longitudinaux opposés du matelas absorbant, entre lesdites deux feuilles, chacun desdites rubans étant fixé à la feuille externe au moins le long de la partie médiane de son bord longitudinal extérieur et le long de ses deux bords transversaux et à la feuille interne le long de ses deux bords longitudinaux opposés et le long de ses deux bords transversaux. Les éléments élastiques sont fixés à l'état allongé sur lesdits rubans, entre les deux bords longitudinaux opposés de ces derniers.

De préférence, les éléments élastiques sont fixés à l'état allongé sur la face des rubans tournée vers la feuille interne.

Par ailleurs, des éléments élastiques supplémentaires peuvent être fixés à la feuille externe imperméable aux liquides.

Les rubans peuvent être disposés soit à l'extérieur des bords longitudinaux opposés du matelas absorbant, soit de manière que les bords longitudinaux intérieurs des rubans soient situés sur la face du matelas tournée vers la feuille interne perméable aux liquides, les rubans chevauchant ainsi les bords longitudinaux opposés du matelas au moins dans la partie médiane de la longueur de ce dernier. Les rubans sont, dans ce cas, de préférence constitués par une matière imperméable aux liquides.

Dans le cas d'une couche-culotte en forme de sablier comprenant deux feuilles en forme de sablier obtenues par découpage d'une échancrure latérale dans chacun des deux bords longitudinaux opposés des feuilles, les rubans portant les éléments élastiques peuvent être disposés par rapport auxdites feuilles de manière que leurs bords longitudinaux extérieurs soient situés à l'extérieur du fond des échancrures latérales des feuilles, de sorte que le découpage des échancrures dans les deux feuilles entraîne également le découpage d'échancrures dans les rubans.

Dans le cas de couches-culottes avec des matelas absorbants en forme de sablier ayant des échancrures dans leurs deux bords longitudinaux opposés, les éléments élastiques sont avantageusement fixés à l'état allongé aux rubans sur une longueur supérieure à la longueur des échancrures du matelas.

Bien qu'il soit possible, dans le cadre de l'invention, de ne fixer qu'un seul élément élastique à chaque ruban, il est avantageux de fixer plusieurs éléments élastiques espacés parallèles sur chaque ruban.

En se référant aux dessins annexés, on va décrire ci-après plus en détail un mode de réalisation illustratif et non limitatif d'une couche-culotte conforme à l'invention; sur les dessins :

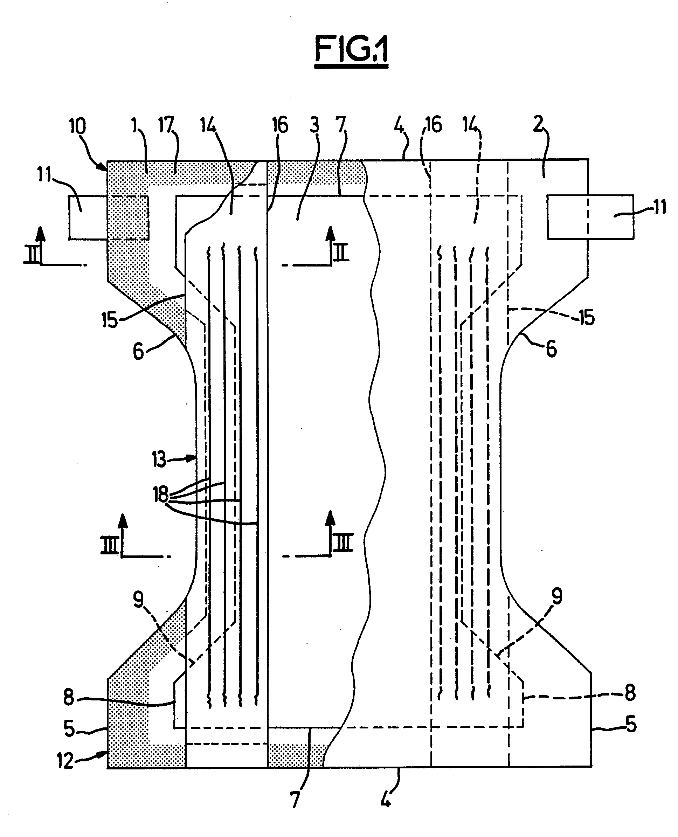

la figure 1 est une vue sur la face interne d'une couche-culotte conforme à l'invention disposée à plat, avec arrachement partiel de la feuille interne perméable aux liquides;

les figures 2 et 3 sont des coupes partielles, à plus grande échelle, suivant II-II et III-III de la figure 1;

la figure 4 est une coupe analogue à celle de la figure 3 montrant la couche-culotte en cours d'utilisation;

les figures 5 et 6 sont des vues analogues à la figure 1 de deux autres modes de réalisation de l'invention;

la figure 7 est une coupe analogue à celle de la figure 3 pour le mode de réalisation de la figure 6.

La couche-culotte à jeter après usage telle qu'illustrée par les dessins comprend une feuille externe 1 imperméable aux liquides, par exemple une feuille de polyéthylène, une feuille interne 2 perméable aux liquides, par exemple une feuille de non-tissé, et un matelas absorbant 3, par exemple à base de pulpe de cellulose défibrée dite "fluff de cellulose", avec incorporation ou non de particules de matière superabsorbante. De tels matelas absorbants sont bien connus et leur structure particulière, la nature des matériaux utilisés, etc. n'entrent pas dans le cadre de la présente invention.

Les deux feuilles 1 et 2 présentent la même forme en sablier, c'est-à-dire une forme générale rectangulaire avec deux bords transversaux opposés 4 rectilignes et deux bords longitudinaux opposés 5 munis chacun d'une échancrure 6 dans la partie médiane de sa longueur.

Le matelas absorbant 3 présente lui aussi une forme en sablier et est disposé en position centrale par rapport aux deux feuilles 1, 2, entre ces dernières, de manière que ses deux bords transversaux 7 rectilignes et ses deux bords longitudinaux 8 munis chacun d'une échancrure 9 sensiblement au milieu de sa longueur se trouvent en retrait par rapport aux bords 4 et 5/6 des feuilles 1, 2.

Au moins la feuille externe 1 est en outre munie, dans la partie arrière 10 plus large de la couche-culotte, située d'un côté des échancrures 6, d'éléments d'attache par adhésif 11 destinées à être fixés, en vue de la fermeture de la couche-culotte autour de l'utilisateur, sur la partie avant 12 plus large de la couche-culotte, la partie intermédiaire 13 dite partie d'entre-jambes, définie par les échancrures 6, étant située entre les cuisses de l'utilisateur.

Un ruban 14 d'une matière souple imperméable aux liquides, par exemple de feuille de polyéthylène s'étend, entre les deux feuilles 1 et 2, sur toute la longueur de la couche-culotte, le long de chaque bord longitudinal du matelas absorbant 3, les rubans étant situés entre le matelas absorbant 3 et la feuille interne 2 perméable aux liquides. Le bord longitudinal extérieur 15 de chaque ruban 14 se trouve décalé légèrement vers l'intérieur par rapport au bord longitudinal 8 correspondant du matelas 3 et décalé légèrement vers l'extérieur par rapport au fond de l'échancrure 6 correspondante des feuilles 1 et 2, de sorte que le découpage des échancrures 6 affecte également légèrement les rubans 14. Le bord longitudinal intérieur 16 de chaque ruban 14 est décalé vers l'intérieur par rapport au fond de l'échancrure 9 correspondante du matelas absorbant 3. Par conséquent, chaque ruban 14 chevauche le bord longitudinal correspondant du matelas absorbant 3 pratiquement sur toute la longueur de l'échancrure 9, c'est-à-dire dans la zone d'entre-jambes.

Les rubans 14 sont fixés à la feuille externe 1 à leurs deux extrémités ainsi que le long de leurs bords longitudinaux extérieurs 15 dans la zone des échancrures 6, c'est-à-dire essentiellement dans leurs parties en contact avec la feuille externe 1. Cette fixation s'effectue par le fait que la feuille externe 1 est munie, sur sa face tournée vers la feuille interne 2, de façon connue en soi d'une application de colle 17 tout autour de la zone recouverte par le matelas 3, en vue de la liaison de la feuille interne 2 à la feuille externe 1 sur le pourtour de matelas 3.

Plusieurs éléments élastiques 18, à savoir quatre éléments élastiques dans l'exemple représenté, sont fixés à l'état allongé sur chaque ruban 14, dans la partie médiane de la longueur du ruban, sur la face du ruban tournée vers la feuille interne 2. Dans l'exemple représenté, deux des éléments élastiques 18 sont situés à l'extérieur et les deux autres à l'intérieur du bord du matelas absorbant 3 défini par le fond de l'échancrure 9 correspondante du matelas. La longueur sur laquelle les éléments élastiques 18 sont fixés aux rubans 14 est supérieure à la longueur des échancrures 9 du matelas 3, de sorte que les éléments élastiques 18 s'étendent non seulement sur la partie d'entre-jambes 13, mais dépassent sur la partie arrière 10 et la partie avant 12 de la couche-culotte.

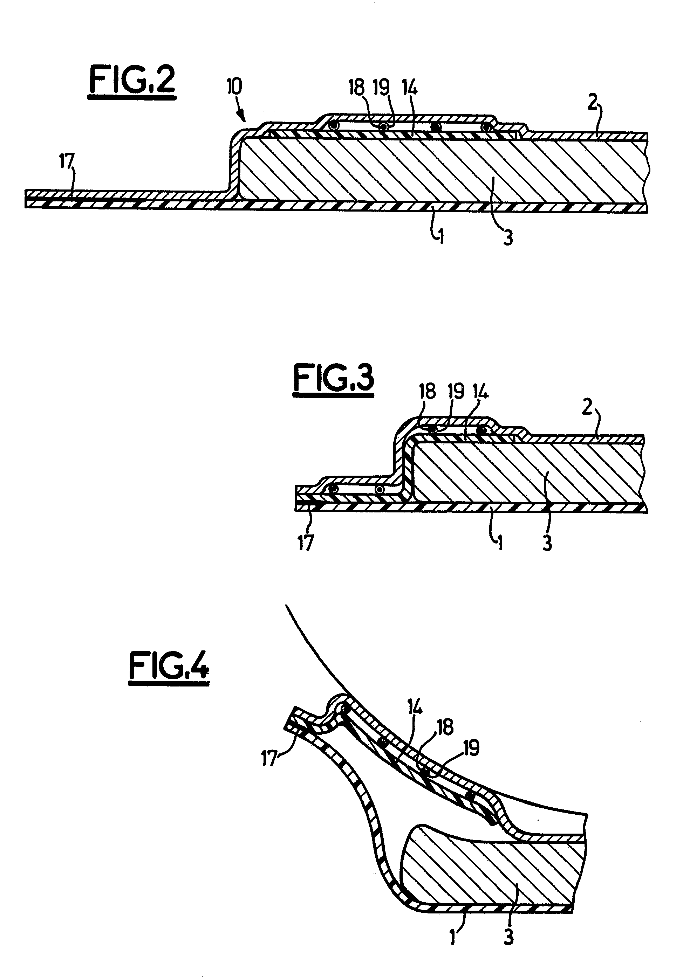

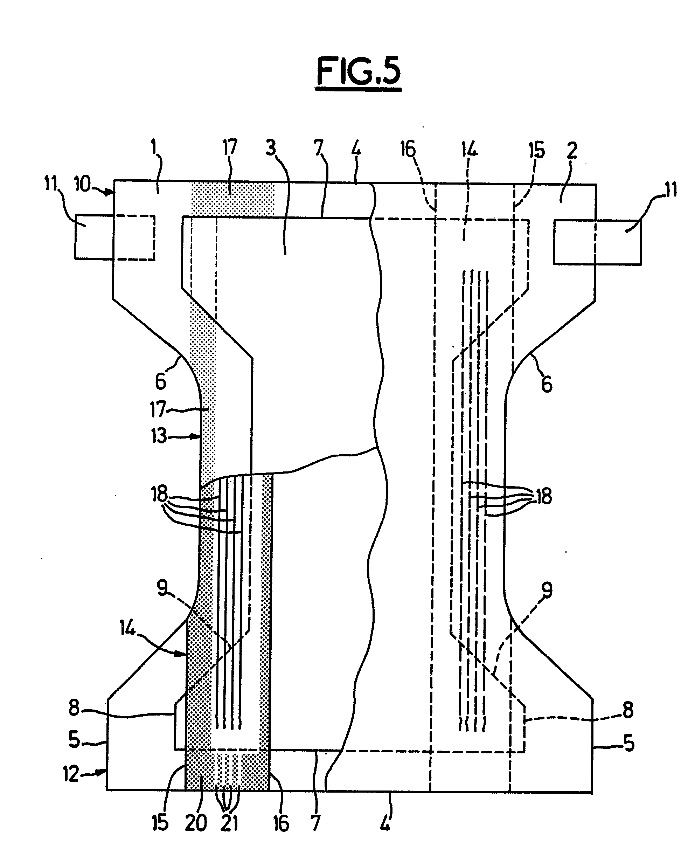

En vue de leur fixation sur les rubans 14, les éléments élastiques 18 sont, de façon connue en soi, enduits de colle sur une longueur correspondant à la longueur de fixation des éléments élastiques à l'état allongé. Selon les figures 2 et 3, les éléments élastiques 18 reçoivent une enduction de colle 19 sur tout leur pourtour, ce qui fait qu'ils adhèrent à la fois aux rubans 14 et à la feuille interne 2. Par conséquent, les éléments élastiques 18 établissent la liaison entre les rubans 14 et la feuille interne 2, de sorte qu'une liaison supplémentaire, par exemple par une ligne de colle, au voisinage du bord longitudinal intérieur 16 des rubans 14, n'est pas absolument nécessaire entre les rubans 14 et la feuille interne 2.

La figure 4 montre l'indépendance des éléments élastiques 18 par rapport au matelas absorbant 3, grâce à leur fixation, non pas sur la feuille externe 1, mais sur les rubans 14 reliés à la feuille externe 1 uniquement le long de leur bord longitudinal extérieur 15 (et à leurs extrémités). Les risques de baillement de la couche-culotte dans la zone d'entre-jambes, sous l'effet des mouvements de l'utilisateur, sont donc pour le moins fortement réduits.

La figure 5 illustre un autre mode de réalisation de l'invention dans lequel chaque ruban 14 portant plusieurs éléments élastiques 18 est fixé à la feuille externe 1 par une encollage 17 en forme de U de cette dernière, cet encollage 17 correspondant aux deux bords transversaux et au bord longitudinal extérieur (15) du ruban 14. Le ruban 14 est à son tour fixé à la feuille interne 2 par un encollage 20, appliquée soit sur le ruban 14, soit de préférence sur la face externe de la feuille interne 2, de manière à s'étendre le long des deux bords longitudinaux opposés 15, 16 et des deux bords transversaux du ruban 14. Il y a lieu de noter que dans ce cas, les éléments élastiques 18 ne sont pas fixés à la feuille interne 2. De plus, on reconnaît sur la figure 5 que l'encollage 20 comporte, à chaque extrémité transversale du ruban 14, des interruptions 21 qui se trouvent dans le prolongement des éléments élastiques 18 et sont destinées à permettre la libre contraction des sections non encollées des éléments élastiques, lors de la fabrication en continu des couches-culottes.

Il est à noter également que dans le mode de réalisation suivant la figure 5, les éléments élastiques 18 au nombre de 4 sont fixés sur chaque ruban 14 de manière à se trouver à l'extérieur du fond de chaque échancrure latérale 9 du matelas absorbant 3, c'est-à-dire à l'extérieur du bord longitudinal du matelas dans la zone d'entre-jambes 13.

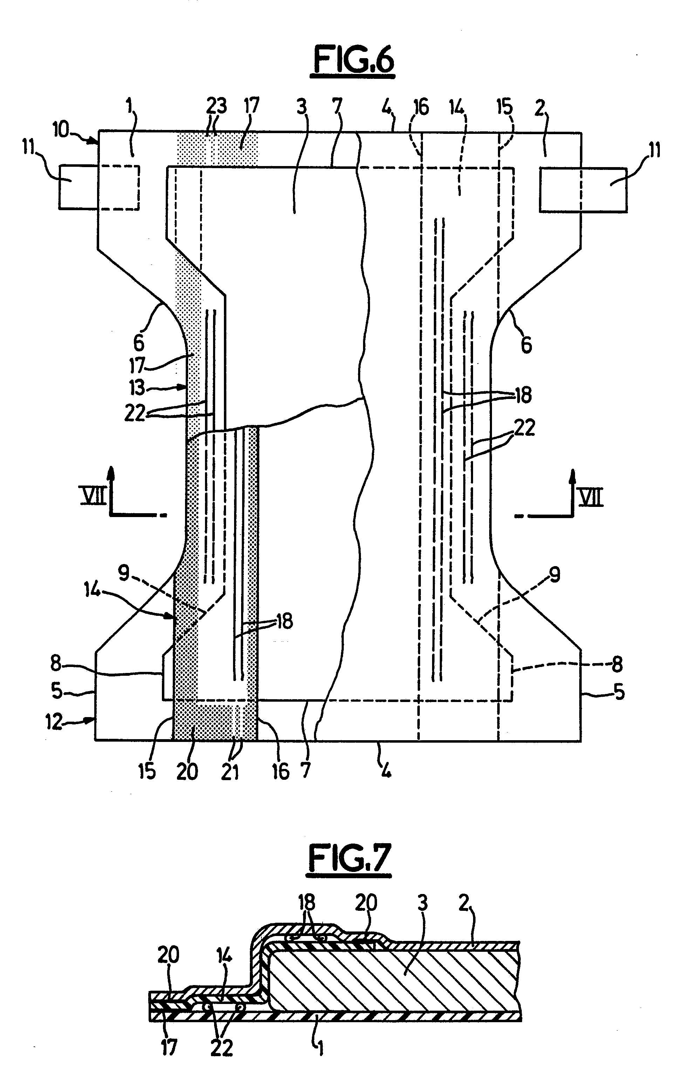

Dans le mode de réalisation préféré de l'invention illustré par les figures 6 et 7, deux éléments élastique longitudinaux 18 sont fixés sur chaque ruban 14, sur la face tournée vers la feuille interne 2, et deux autres éléments élastiques longitudinaux 22 sont fixés directement sur la feuille externe 1, entre le fond de l'échancrure 9 du matelas 3 et le fond de l'échancrure 6 des feuilles 1 et 2. Il est à noter que les éléments élastiques 22 fixés à la feuille externe 1 sont plus courts que les éléments élastiques 18 fixés au ruban 14, leur longueur n'étant pas supérieure à la longueur du fond de l'échancrure 9 du matelas 3 (zone d'entre-jambes 13). Par contre, les éléments élastiques 18 sont fixés sur les rubans sur une longueur qui est presque ou sensiblement égale à la longueur du matelas absorbant 3.

Pour permettre la libre rétraction des sections non encollées des éléments élastiques 22, l'encollage 17 en U prévu sur la feuille externe 1 pour la fixation de chaque ruban 14 à la feuille externe 1 suivant ses deux bords transversaux et son bord longitudinal extérieur comporte ici, dans chacune de ses deux parties transversales, deux interruptions 23 alignées avec les éléments élastiques 22.

Les deux éléments élastiques 18 fixés par collage uniquement sur la face de chaque ruban 14 tournée vers la feuille interne 2 et non pas sur la feuille interne 2 sont disposés sur le ruban 14 de manière à se trouver décalés vers l'intérieur par rapport au fond de l'échancrure 9 correspondante du matelas 3. Pour permettre la libre contraction des sections non encollées des éléments élastiques 18, l'encollage 20 prévu sur le ruban 14 ou sur la feuille interne 2 pour la fixation du ruban 14 à la feuille interne 2 le long de ses deux bords longitudinaux opposés 15, 16 et de ses deux bords transversaux opposés comporte, dans les parties transversales, des interruptions 21 situées dans le prolongement des éléments élastiques 18.

Il y a lieu de noter que les modes de réalisation représentés et décrits n'ont été donnés qu'à titre d'exemples illustratifs et non limitatifs et que de nombreuses modifications et variantes sont possibles dans le cadre de l'invention.

Ainsi, le nombre des éléments élastiques 18 fixés sur les rubans 14 (de même que le nombre des éléments élastiques 22 fixés sur la feuille externe) peut varier entre 1 et 4 ou même davantage. La disposition des éléments élastiques par rapport aux bords longitudinaux du matelas (par rapport au fond des échancrures latérales dans le cas de matelas en forme de sablier) est également variable, les éléments élastiques pouvant être disposés à l'extérieur, à l'intérieur ou en partie à l'extérieur et en partie à l'intérieur desdits bords.

La largeur des rubans 14 est également variable.

Il est également possible de placer les rubans 14 portant les éléments élastiques 18, non pas à cheval sur les bords longitudinaux de matelas 3, mais complètement à l'extérieur du matelas, au moins dans la zone d'entre-jambes 13.

Il est par ailleurs possible de donner aux rubans 14 une largeur telle que leur bord longitudinal extérieur 15 soit décalé vers l'extérieur par rapport au bord longitudinal 8 du coussin absorbant 3, sur toute la longueur de ce dernier. Dans ce cas, les rubans 14 sont avantageusement reliés, sur toute leur longueur, à la feuille externe 1 (encollage 17) au voisinage de leur bord longitudinal extérieur, ce qui améliore l'effet de barrage d'étanchéité des rubans 14. Cela est valable en particulier pour des matelas absorbants avec des échancrures latérales peu profondes, ou pour des matelas rectangulaires sans échancrures latérales.

Ce qui importe, dans le cadre de l'invention, est que les éléments élastiques 18 aient une indépendance optimale par rapport à la feuille externe 1 et par rapport au matelas absorbant 3 fixé sur la feuille externe 1, de manière à rester toujours plaqué contre les cuisses et assurer ainsi toujours une barrière efficace, notamment pour la rétention des matières fécales.

Par ailleurs, l'invention est bien entendu applicable également à des couches-culottes et à des articles d'hygiène analogues pour enfants et pour adultes incontinents, n'ayant pas une forme en sablier et/ou comportant des matelas absorbants qui ne sont pas en forme de sablier.

## Revendications

1. Couche-culotte du type à jeter après usage comprenant une feuille externe (1) imperméable aux liquides, une feuille interne (2) perméable aux liquides, les deux feuilles de forme allongée ayant pratiquement les mêmes dimensions et étant superposées, un matelas absorbant (3) de forme allongée ayant une longueur et une largeur plus faible que celles desdites feuilles et disposé entre lesdites feuilles de manière que ses bords (7, 8/9) soient en retrait par rapport aux bords (4, 5/6) desdites feuilles, les deux feuilles étant reliées entre elles autour du matelas, des éléments élastiques (18) s'étendant à l'état allongé le long des bords longitudinaux opposés du matelas absorbant, et des moyens (11) de fermeture de la couche-culotte autour de la taille de l'utilisateur, caractérisée par le fait qu'elle comprend, en outre, deux rubans longitudinaux (14) s'étendant à l'état tendu sur toute la longueur de la couche-culotte, le long des bords longitudinaux opposés du matelas absorbant, entre lesdites deux feuilles, chacun desdits rubans étant fixé à la feuille externe (1) au moins le long de la partie médiane de son bord longitudinal extérieur (15) et le long de ses deux bords transversaux et à la feuille interne (2) le long de ses deux bords longitudinaux opposés (15 et 16) et le long de ses deux bords transversaux, et que les éléments élastiques (18) sont fixés à l'état allongé sur lesdits rubans, entre les deux bords longitudinaux opposés (15, 16) de ces derniers.

2. Couche-culotte suivant la revendication 1, caractérisée par le fait que les éléments élastiques (18) sont fixés à l'état allongé sur la face des rubans (14) tournée vers la feuille interne (2).

3. Couche-culotte suivant la revendication 2, caractérisée par le fait que des éléments élastiques supplémentaires sont fixés à la feuille externe (1) imperméable aux liquides.

4. Couche-culotte suivant l'une quelconque des revendications précédentes, caractérisée par le fait que les rubans (14) sont disposés par rapport aux bords longitudinaux opposés (8/9) du matelas absorbant (3) de manière que les bords longitudinaux intérieurs (16) des rubans soient situés sur la face du matelas tournée vers la feuille interne (2), les rubans chevauchant ainsi les bords longitudinaux opposés du matelas au moins dans la partie médiane de la longueur de ce dernier.

5. Couche-culotte suivant la revendication 4, caractérisée par le fait que les rubans (14) sont constitués par une matière imperméable aux liquides.

6. Couche-culotte suivant l'une quelconque des revendications précédentes, en forme de sablier, comprenant deux feuilles (1, 2) en forme de sablier obtenues par découpage d'une échancrure latérale (6) dans chacun des deux bords longitudinaux opposés (5) des feuilles, caractérisée par le fait que les rubans (14) portant les éléments élastiques (18) sont disposés par rapport auxdites feuilles de manière que leurs bords longitudinaux extérieurs (15) soient situés à l'extérieur du fond des échancrures latérales (6) des feuilles, de sorte que le découpage des échancrures dans les feuilles entraîne également le découpage d'échancrures dans les rubans.

7. Couche-culotte suivant l'une quelconque des revendications précédentes, comprenant un matelas absorbant en forme de sablier ayant des échancrures (9) dans ses deux bords longitudinaux opposés (8), caractérisée par le fait que les éléments élastiques (18) sont fixés à l'état allongé aux rubans (14) sur une longueur supérieure à la longueur des échancrures (9) du matelas.

8. Couche-culotte suivant l'une quelconque des revendications précédentes, caractérisée par le fait que les zones de collage (20) prévues pour la fixation des rubans (14) à la feuille interne (2) comportent des interruptions (21) dans le prolongement des éléments élastiques (18) fixés sur les rubans (14).

# FIG.1

## FIG.2

## FIG.3

## FIG.4

# FIG.5

## FIG.6

## FIG.7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y,D | EP-A-0149999 (BOUSSAC SAINT FRERES B.S.F.) <br> * revendications 1-10; figures 3, 4 * | 1 | A61F13/15 |
| A | | 2-8 | |
| | --- | | |
| Y,D | EP-A-0270979 (BOUSSAC SAINT FRERES B.S.F.) <br> * colonne 9, ligne 48 - colonne 10, ligne 15; revendications 1-4; figures 5-7 * | 1 | |
| A | | 2-8 | |
| | --- | | |
| A,D | EP-A-0219326 (THE PROCTER & GAMBLE COMPANY) <br> * figures 1-7 * | 1 | |
| | --- | | |
| A,D | GB-A-2188532 (UNI-CHARM CORPORATION) <br> * figures 1-18 * | 1 | |
| | ----- | | |

**DOMAINES TECHNIQUES RECHERCHES (Int. Cl.5)**

A41B

Le présent rapport a été établi pour toutes les revendications

| Lieu de la recherche | Date d'achèvement de la recherche | Examinateur |
|---|---|---|
| LA HAYE | 14 FEVRIER 1990 | KARIPIDOU C. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
.............................................................
& : membre de la même famille, document correspondant

EPO FORM 1503 03.82 (P0402)